# EUROPEAN PATENT SPECIFICATION

(11) **EP 1 642 575 B1**
(45) Date of publication and mention of the grant of the patent: **28.10.2009**
(21) Application number: 05026505.7
(22) Date of filing: 04.03.1998
(51) Int. Cl.: A61K 31/19, A61P 25/28

(54) **Use of r-carprofen for the prevention of alzheimer's disease**
Verwendung von r-carprofen zur Vorbeugung der Alzheimerschen Krankheit
Utilisation de r-carprofen pour la prévention de la maladie d'alzheimer

(30) Priority: 10.03.1997 US 814490
(43) Date of publication of application: 05.04.2006
(62) Divisional of application: 98908904.0
(73) Proprietor: LOMA LINDA UNIVERSITY MEDICAL CENTER, Loma Linda, CA 92350 (US)
(72) Inventor: Wechter, William, J., Ojai, CA 93023 (US); Mccracken, John, D., Redlands, CA 92373 (US)
(74) Representative: Dzieglewska, Hanna Eva

(56) References cited:
- WO-A-93/16689
- WO-A-93/24115
- WO-A-96/28148
- US-A- 5 331 000
- US-A- 5 382 591
- BREITNER J C S: "Inflammatory processes and antiinflammatory drugs in Alzheimer's disease: A current appraisal" NEUROBIOLOGY OF AGING 1996 UNITED STATES, vol. 17, no. 5, 1996, pages 789-794, XP002365660 ISSN: 0197-4580
- W.J. WECHTER: "Rac-flurbiprofen is more ulcerogenic than its (S)-enantiomer." CHIRALITY, vol. 5, no. 7, 1993, pages 492-494, XP000600914
- STEWART WALTER F ET AL: "Risk of Alzheimer's disease and duration of NSAID use" NEUROLOGY, vol. 48, no. 3, 1 March 1997 (1997-03-01), pages 626-632, XP009060951 ISSN: 0028-3878

## Description

### Field of the Invention

The present invention relates to a medical use useful in prevention or delay of onset of symptoms of Alzheimer's Disease.

### Background of the Invention

Many NSAIDs exhibit molecular chirality, and thus have R- and S-enantiomers. Such compounds typicatty are produced as racemic mixtures, which can subsequently be separated into the individual enantiomers.

The enantiomers of several 2-arylpropionic acid NSAIDs are discussed in Yamaguchi et al., Nippo Yakurigaku Zasshi, 90:295-302 (1987). Yamaguchi *et al.* state that the S-enantiomers of 2-arylpropionic acids have 15-300 times higher prostaglandin synthetase inhibitory activities than the R-enantiomers in the rat.

Caldwell et al., Biochem. Pharmacol. 37: 105-114 (1988) allege that "at best, the R-isomers [of 2-arylpropionic acids] function as prodrugs for the therapeutically active S-forms" when the racemic drug is administered and thus add to both in the therapeutic and toxic effects of the active S-enantiomers. Caldwell *et al.* further contend that "at worst, the R-enantiomers are undesirable impurities in the active drug" causing difficulties due to non-stereoselective toxicity. The authors indicate that the use of the S-isomers alone should provide safer and more effective use of this class of drugs.

Similarly, it has been generalized that the pharmacokinetics of the enantiomers of 2-arylpropionic acids are different due, at least in part, to the unidirectional metabolic inversion of the R- to the S-enantiomer. However, it has been found that this interconversion depends on the particular compound and the particular species in which it is administered. Jamali, Eur. J. Drug Metabolism Pharmaco. 13: 1-9(1988).

Because of the toxicity and side effects previously described, many NSAIDs are no longer in use in human medicine as analgesics. Some of these NSAIDs include tiaprofenic acid, suprofen, carprofen, pirprofen and indoprofen.

A disease for which effective treatment is needed is Alzheimer's Disease (AD) which is a degenerative brain disorder associated with extensive loss of specific neuronal subpopulations and characterized clinically by progressive loss of memory, cognition, reasoning, judgment and emotional stability that gradually leads to profound mental deterioration and ultimately death. AD is a common cause of progressive mental failure (dementia) in aged humans and is believed to represent the fourth most common medical cause of death in the United States. AD has been observed in varied races and ethnic groups worldwide and presents a major present and future public health problem. The disease is currently estimated to affect up to four million individuals in the United States alone. To date, AD has proven to be incurable, and presently causes up to 100,000 deaths yearly.

The brains of individuals with AD exhibit neuronal degeneration and characteristic lesions variously referred to as amyloidogenic plaques, vascular amyloid angiopathy, and neurofibrillary tangles. Large numbers of these lesions, particularly amyloidogenic plaques and neurofibrillary tangles, are generally found in several areas of the human brain important for memory and cognitive function in patients with AD. Smaller numbers of these lesions in a more restricted anatomical distribution are found in the brains of most aged humans who do not have clinical AD, as well as patients suffering from Down's Syndrome and Hereditary Cerebral Hemorrhage with Amyloidosis of the Dutch-Type.

It is presently believed that progressive cerebral deposition of particular amyloidogenic proteins, beta -amyloid proteins ( beta AP), play a seminal role in the pathogenesis of AD and can precede cognitive symptoms by years or decades. Recently, it has been shown that beta AP is released from neuronal cells grown in culture and is present in cerebrospinal fluid (CSF) of both normal individuals and AD patients.

A possible correlation to the plaque pathology has been developed by several groups demonstrating the direct beta AP neurotoxicity toward cultured neurons. More recently, in addition to the direct neurotoxicity, an inflammatory response in the AD brain, perhaps elicited by beta AP, also contributes to the pathology of the disease. A limited clinical trial with the NSAID indomethacin exhibited a retardation in the progression of Alzheimer's dementia (Rogers et al., Science, 260:1719-1720 (1993)).

Previous methods of treating AD are disclosed, for example, in U.S. Patent No. 5,576,353 (use of N-propargyl-aminoindan compounds) and U.S. Patent No. 5,552,415 (use of raloxifene and related compounds). A continuing need exists for effective methods for preventing, delaying, and treating AD.

### Summary of the Preferred Embodiments

As set forth in the accompanying claims, a further medical use of R-carprofen is provided for preventing or delaying the onset of Alzheimer's Disease. A composition for such use comprises an effective Alzheimer's Disease prophylactic amount of enantiomerically stable R-carprofen or a pharmaceutically acceptable salt thereof. The composition is substantially free of the S-enantiomer of said R-NSAID.

In accordance with yet a further aspect of the present invention, there is provided a composition comprising an effective Alzheimer's Disease prophylactic amount of an enantiomerically stable R-carprofen or a pharmaceutically acceptable salt thereof, said composition being substantially free of the S-enantiomer of said R-NSAID.

### Detailed Description of the Preferred Embodiments

The inventive compositions comprise at least the enantiomerically stable R-carprofen and are substantially free of the corresponding S-carprofen. As used herein, the term "enantiomerically stable" means that at steady state there is no more than about 20% of the circulation NSAID as its S-enantiomer and preferably no more than 10% (i.e., 90% R, 10% S). A suitable measure of this ratio is obtained by evaluating the relative concentrations of the two enantiomers in the blood plasma or urine vs. time.

The rate of change of enantiomer concentration in plasma, for example, is assumed to reflect quantitatively the change in drug concentrations throughout the body. This rate can be approximated by first-order kinetics. See Gibaldi et al. Pharmacokinetics, (1982) Chapter 1, pp. 1-5.

Pharmacokinetic data and an explanation of the present state of knowledge for many NSAIDs are presented in Jamali, "Pharmacokinetics of Enantiomers of Chiral Non-steroidal Anti-inflammatory Drugs, " Eur. J. Drug Metab. Pharmacokin. (1988), Vol. 13, No. 1, pp. 1-9, which is hereby incorporated by reference.

The term "substantially free" indicates that the amount of S-NSAID, if any, present in the composition is insufficient to elicit an adverse effect in the patient to whom the composition is administered or, at most elicits an adverse effect that is tolerable to the patient and is outweighed by the beneficial effect or effects. Preferably, the inventive composition contains at least 90% by weight of a R-NSAID and 10% by weight or less of the corresponding S-NSAID, based upon the total amount of NSAID present in the composition. That is, the ratio of R-NSAID to S-NSAID in the composition is at least about 90:10. Particularly preferably, the inventive composition contains at least 99% by weight of the R-NSAID and 1 % or less of the corresponding S-NSAID.

The chemical structures of NSAIDs vary. Certain NSAIDs, such as ketoprofen and flurbiprofen are arylpropionic acids, while others are cyclized derivatives of arylpropionic acids, arylacetic acids, thiazinecarboxamides, etc.. Depending on the structure of a particular NSAID, the compound may or may not exhibit chirality, *i.e.* may not have R- and S-enantiomers.

Some of the NSAIDs related to the present invention are:

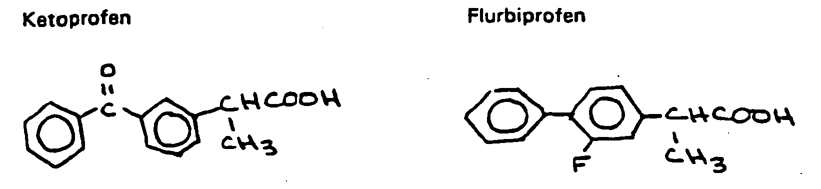

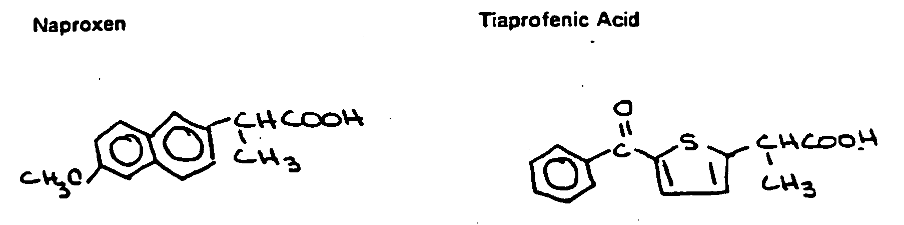

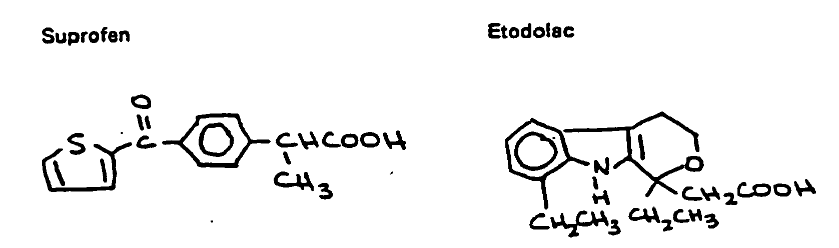

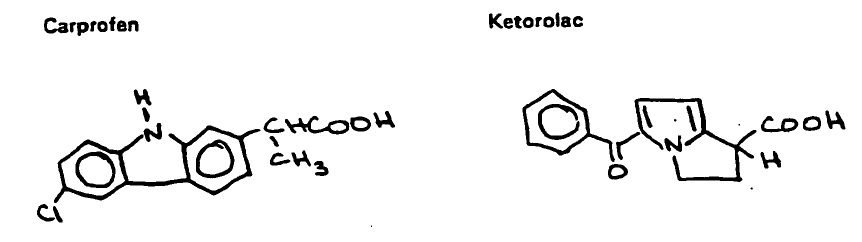

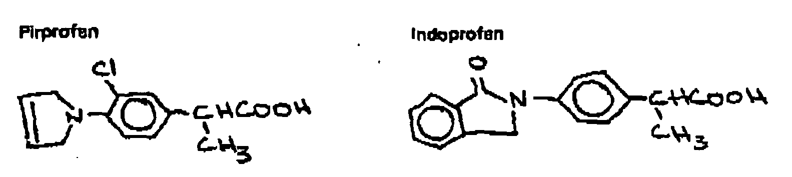

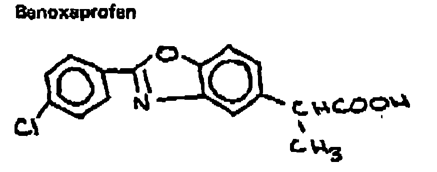

The R-NSAID employed according to the present invention is an arylpropionic acid, in particular R-carprofen. NSAIDs have been used in human medicine in the U.S. and/or Europe as racemates, with the exception of naproxen which is commercially available as the S-isomer only, and are enantiomerically stable. Enantiomerically unstable NSAIDs, for example propionic acid derivatives such as ibuprofen, are not encompassed by the present invention.

Descriptions of specific NSAIDs can be found in various publications. Ketoprofen, for example, is described in U.S. Patent No. 3,641,127. A description of flurbiprofen is found in U.S. Patent No. 3,755,427. Ketorolac, another chiral NSAID, is described in U.S. Patent No. 4,089,969.

A large number of NSAIDs are commercially available either in the form of racemic mixtures or as optically pure enantiomers, In all cases racemic mixtures contain equal amounts of the R- and S-isomers of the NSAID are provided. For example, the following racemates can be obtained through Sigma Chemical Co.: ketoprofen, flurbiprofen, etodolac, suprofen, carprofen, indoprofen and benoxaprofen. Naproxen, marketed as the S-isomer only, is also available from this source. Additionally, many commercial sources exist for the stereospecific R-isomers of many NSAIDs. R-ketoprofen, R-flurbiprofen and R-ketorolac, for example, are available through Sepracor, Inc.; R-naproxen can be obtained as the sodium salt through Sigma Chemical Co.; R-etodolac is available from Wyeth-Ayerst; R-tiaprofenic acid is available through Roussel (France, Canada, Switzerland, Spain, Denmark, Italy); R-suprofen is manufactured by McNiel Pharmaceuticals; R-carprofen is available from Roche; R-pirprofen is available through Ciba (France, Belgium, Denmark); R-indoprofen can be obtained through Carlo Elba (Italy, U.K.); and R-benoxaprofen is manufactured by Eli Lilly Co..

In addition to commercial sources, racemic mixtures of NSAIDs can be produced by methods described in numerous references and U.S. Patents. Synthesis of ketoprofen, for example, is described in U.S. Patent No. 3,641,127, while the synthesis of racemic ketorolac is disclosed in Muchowski et al., J. Med. Chem., 28(8):1037-1049 (1985). The optically pure R-isomers of the selected NSAIDs can then be obtained by resolving the racemic mixtures according to well-known methods. See, *e.g.,* U.S. Patent No. 5,331,000 (R-ketoprofen) and U.S. Patent No. 5,382,591 (R-ketorolac).

In general, the total daily dose range for R-carprofen for the condition described herein, is from about 0.1 mg to about 2000 mg, in single or divided doses.

In managing the patient, the therapy should be initiated at a lower dose, perhaps about 0.1 mg to about 100 mg and increased up to about 1000 mg or higher depending on the patient's global response. It is further recommended that infants, children, patients over 65 years, and those with impaired renal or hepatic function, initially receive low doses, and that they be titrated based on individual response(s) and blood level(s).

It may be necessary to use dosages outside these ranges in some cases as will be apparent to those skilled in the art. Further, it is noted that the ordinary skilled clinician or treating physician will know how and when to interrupt, adjust or terminate therapy in consideration of individual patient response.

Any suitable route of administration may be employed for providing the patient with an effective dosage of a R-NSAID. For example, oral, rectal, transdermal, parenteral (subcutaneous, intramuscular, intravenous), intrathecal, and like forms of administration may be employed. Dosage forms include tablets, troches, dispersions, suspensions, solutions, capsules or patches.

In order to aid in patient compliance with daily dosage requirements, the R-NSAIDs may also be administered by formulating them in a toothpaste. The drug is dissolved in an ethyl alcohol solution and added to the toothpaste so that the final concentration of R-NSAID is from about 0.01 to about 1 % on a weight compositions of the present invention basis.

The pharmaceutical compositions of the present invention comprise a R-carprofen, or a pharmaceutically acceptable salt thereof, as the active ingredient and may also contain a pharmaceutically acceptable carrier, and optionally, other therapeutic ingredients.

The terms "pharmaceutically acceptable salts" or "a pharmaceutically acceptable salt thereof" refer to salts prepared from pharmaceutically acceptable, non-toxic acids or bases. Suitable pharmaceutically acceptable salts include metallic salts, e.g. salts of aluminum, calcium, lithium, magnesium, potassium, sodium and zinc or organic salts, e.g. salts of lysine, N,N'-dibenzylethylenediamine, chloroprocaine, choline, diethanolamine, ethylenediamine, meglumine (N-methylglucamine), procaine and tris.

The term "with reduced gastrointestinal toxicity" as used herein means that the administration of the particular R-NSAID is less ulcerogenic to the gastrointestinal tract of the human or other mammal than the corresponding racemate or S-NSAID. One measure of ulcerogenic activity is the small bowel ulcer score. A rat is treated daily through oral administration of the R-NSAID for 30 days. At the end of the 30 days, the rat is sacrificed and the intestines removed. Lesions of appreciable size in the mucosa are measured. A cumulative score equaling the sum of the diameters of the ulcers measured are reported as the ulcer score. An ulcer score essentially equal to that of a control rat, or a reduction of the ulcer score of at least 50 to 90%, preferably at least 80%, as compared to the corresponding S-NSAID or racemate, is considered a reduction in gastrointestinal toxicity.

The compositions of the present invention can be prepared in any desired form, for example, tablets, powders, capsules, suspensions, solutions, elixirs, and aerosols. Carriers such as starches, sugars, microcrystalline cellulose, diluents, granulating agents; lubricants, binders, disintegrating agents, and the like may be used in the cases of oral solid preparations. Oral solid preparations (such as powders, capsules, and tablets) are preferred over oral liquid preparations. The most preferred oral solid preparations are tablets. If desired, tablets may be coated by standard aqueous or nonaqueous techniques.

In addition to the common dosage forms set out above, the compounds of the present invention may also be administered by controlled release means and/or delivery devices such as those described in U.S. Pat. Nos.: 3,845,770; 3,916,899; 3,536,809; 3,598,123; and 4,008,719.

Pharmaceutical compositions of the present invention suitable for oral administration may be presented as discrete units such as capsules, cachets, or tablets, or aerosol sprays, each containing a predetermined amount of the active ingredient, as a powder or granules, or as a solution or a suspension in an aqueous liquid, a non-aqueous liquid, an oil-in-water emulsion, or a water-in-oil liquid emulsion. Such compositions may be prepared by any of the conventional methods of pharmacy, but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation.

For example, a tablet may be prepared by compression or molding, optionally, with one or more accessory ingredients. Compressed tablets may be prepared by compressing in a suitable machine the active ingredient in a free-flowing form such as powder or granules, optionally mixed with a binder, lubricant, inert diluent, surface active or dispersing agent. Molded tablets may be made by molding, in a suitable machine, a mixture of the powdered compound moistened with an inert liquid diluent. Desirably, each tablet contains from about 0.1 mg to about 1000 mg of the active ingredient, and each cachet or capsule contains from about 0.1 mg to about 600 mg of the active ingredient. Most preferably, the tablet, cachet or capsule contains either one of four dosages, about 0.1 mg, about 50 mg, about 100 mg and about 200 mg of the active ingredient.

It has surprisingly been discovered that administration of an enantiomerically stable R-carprofen appears to prevent or delay the onset of Alzheimer's Disease, without attendant COX-mediated toxicity.

Thus, in accordance with the present invention, patients at risk of developing Alzheimer's Disease patients are treated with R-carprofen at high dose, that is, at an effective Alzheimer's Disease prophylactic amount. As used herein, an "effective Alzheimer's Disease prophylactic amount" is that amount which will delay the onset of symptoms of AD by at least 6 months. More specifically, a preferred effective AD prophylactic amount will be within the range from about 50 to 2000 mg of R-carprofen per day, the amount again being preferably administered in a divided dose based on the plasma half-life of R-carprofen.

The examples are merely for reference purposes.

### EXAMPLE 1: Chemoprotective Effect and Toxicity of R-Flurbiprofen

A study was performed to compare the R- and S-isomers of flurbiprofen with regard to their effect on the Labelling Index (LI) and duodenal ulceration in contrast to the S-isomer.

Female Sprague-Dawley rats were randomized to 4 groups (N =10) receiving 6.3 mg/Kg/day R-flurbiprofen; 6.3 mg/Kg/day S-flurbiprofen; 12.5 mg/Kg/day racemic flurbiprofen; or vehicle control. Fasted rats were sacrificed after 30 days. Small bowel ulcer score was recorded in each group.

The LI is calculated using a histologic biomarker of proliferating cells, a monoclonal antibody to Bromo-deoxyuridine (BrD-U). Intestinal crypts are examined microscopically in longitudinal sections such that proliferating cells are identified and quantified as a proportion of total crypt cells. An U was determined for each rat using BrD-U staining to identify the proportion of mitotic cells in the crypt of Leiberkuhn. Twelve well-oriented crypts (distal colon) were examined in each rat.

The small bowel ulcer score was 0.05; 0.62; 4.54; and 3.22 in the control, R-flurbiprofen, S-flurbiprofen and racemic flurbiprofen groups, respectively. The **LI** was 12.62 in control animals. The LI was reduced to 8.71 and 9.09 in the R- and S-flurbiprofen treated animals, (P< 0.05) and further reduced in animals receiving equal-molar doses of both enantiomers.

The results of this study indicate that R-flurbiprofen is much less ulcerogenic than its S-enantiomer, yet suppresses cell proliferation in the distal colon, a chemopreventive effect.

### EXAMPLE 2: Toxicity of R-Etodolac

The effects of the isomers of etodolac in the guinea pig are determined as follows. Groups of 6-10 guinea pigs are dosed orally with either vehicle, racemic etodolac (2, 10. 5, 1 and 0.2 mg/kg), S-etodolac (20, 10, 5, 1 and 0.1 mg/kg), or R-etodolac (2, 10, 5, 1 and 0.1 mg/kg). Within 24 hours after the dose, the animals are euthanized and gross abnormalities are recorded in the GI tracts, with particular attention to the gastric mucosa of the stomach. Microerosions and redness (irritations) are noted, and the effects are compared between the treatment groups as described by Abert & Larsson (Acta Pharmacol. Toxicol. 28: 249-257, 1970). Based on such observations, the R-isomer is seen to cause virtually no gastrointestinal irritation.

### EXAMPLE 3: Inhibitory Effect on the Activity of Cyclooxygenase

Cyclooxygenase inhibitors (for example aspirin and indomethacin) are known to cause damage and irritation of the gastric mucosa. Assays to determine the inhibitory effect of R-, S- and racemic ketoprofen, reference agents and vehicles on cyclooxygenase activity are conducted using RBL-1 cells (rat basophilic leukemia cell line). The effects of the test compounds, reference agents or vehicles are assessed on the cyclooxygenase-mediated production of PGF_{2α}.

RBL-1 cells are grown in culture in Eagle's minimum essential medium supplemented with 12% fetal bovine serum and 1:100 antibiotic/antimycotic mixture at 27° C. Cells are harvested via centrifugation, washed with cold phosphate buffered saline (PBS), and suspended in PBS supplemented with 0.88 µM CaCl₂. Cells are incubated in the presence of a screening concentration of that compound or reference agent. Alternatively, cells are incubated in the presence of a vehicle.

Following the incubation period, cyclooxygenase activity is stimulated by the addition of 5 *µ*M of a calcium ionophore to the incubation medium. The reaction is terminated by chilling the tubes on ice.

The cells are then separated via centrifugation, and the supernatant is removed. Aliquots of the supematant are used to measure the calcium-ionophore-stimulated production of PGF_{2α} via radioimmunoassay.

For each experiment, a vehicle-control is evaluated. A reference standard is also evaluated at a single concentration with each assay.

The results from the aforementioned studies indicate hat R-NSAIDs are safe alternatives for chemoprophylaxis in colon cancer. R-NSAIDs suppress cell proliferation in the distal colon, an anti-neoplastic effect.

## Claims

1. Use of enantiomerically stable R-carprofen or a pharmaceutically acceptable salt thereof for the manufacture of a medicament for preventing or delaying the onset of symptoms of Alzheimer's Disease, said medicament being substantially free of the S-enantiomer of said R-carprofen.

2. The use of Claim 1, wherein the ratio of said R-carprofen to said S-carprofen in said medicament is at least 90:10 by weight.

3. The use of Claim 1 or 2, wherein the ratio of said R-carprofen to said S-carprofen is at least 99:1 by weight.

4. The use of any one of Claims 1 to 3, wherein said pharmaceutically acceptable salt of said R-carprofen is a metal salt or an organic salt.

5. The use of Claim 4, wherein said R-carprofen metal salt is selected from the group consisting of a sodium, potassium, calcium, magnesium, lithium, aluminium, and zinc salt, or wherein said R-carprofen organic salt is selected from the group consisting of a lysine, N,N'-bibenzylethylenediamine, chloroprocaine, choline, diethanolamino, ethylenediamine, meglumine, procaine and tris salt,

6. The use of any one of Claims 1 to 5, wherein each does of said medicament comprises about 0.1 mg to 2000 mg of said R-carprofen or salt.

7. The use of Claim 4 or 5, wherein each dose of said medicament comprises about 1 mg to 600 mg of said R-carprofen or salt.

8. The use of any one of Claims 1 to 7, wherein said medicament further comprises a pharmaceutically acceptable carrier.

9. The use of any one of Claims 1 to 8, wherein said medicament is in a form suitable for administration orally, transdermally, intravenously, or intrathecally.

10. An enantiomerically stable R-carprofen or a pharmaceutically acceptable salt thereof for use in preventing or delaying the onset of symptoms of Alzheimer's Disease, wherein said R-carprofen or salt thereof is provided as a medicament being substantially free of the S-enantiomer of said R-carprofen.

11. The R-carprofen of claim 10, wherein said medicament is as defined in any one of Claims 2 to 9.

## Patentansprüche

1. Verwendung von enantiomerstabilem R-Carprofen oder eines pharmazeutisch verträglichen Salzes davon zur Herstellung eines Arzneimittels zur Vorbeugung oder Verzögerung des Auftretens von Symptomen der Alzheimer Krankheit, wobei das Arzneimittel im Wesentlichen kein S-Enantiomer von R-Carprofen enthält.

2. Verwendung nach Anspruch 1, wobei das Gewichtsverhältnis von R-Carprofen zu S-Carprofen im Arzneimittel mindestens 90:10 beträgt.

3. Verwendung nach Anspruch 1 oder 2, wobei das Gewichtsverhältnis von R-Carprofen zu S-Carprofen im Arzneimittel mindestens 99:1 beträgt.

4. Verwendung nach einem der Ansprüche 1 bis 3, wobei das pharmazeutisch verträgliche R-Carprofensalz ein Metallsalz oder ein organisches Salz ist.

5. Verwendung nach Anspruch 4, wobei das Metallsalz des R-Carprofens ausgewählt ist aus der Gruppe bestehend aus einem Natrium-, Kalium-, Calcium-, Magnesium-, Lithium-, Aluminium- und Zinksalz, oder wobei das organische R-Carprofensalz ausgewählt ist aus der Gruppe bestehend aus einem Lysin-, N,N'-Dibenzylethylendiamin-, Chlorprocain-, Cholin-, Diethanolamin-, Ethylendiamin-, Meglumin-, Procain- und Trissalz.

6. Verwendung nach einem der Ansprüche 1 bis 5, wobei jede Dosis des Arzneimittels etwa 0.1 mg bis 2000mg R-Carprofen oder dessen Salz enthält.

7. Verwendung nach einem der Ansprüche 4 oder 5, wobei jede Dosis des Arzneimittels etwa 1 mg bis 600mg R-Carprofen oder dessen Salz enthält.

8. Verwendung nach einem der Ansprüche 1 bis 7, wobei das Arzneimittel zusätzlich einen pharmazeutisch verträglichen Träger enthält.

9. Verwendung nach einem der Ansprüche 1 bis 8, wobei das Arzneimittel in einer zur oralen, transdermalen, intravenösen oder intrathekalen Verabreichung geeigneten Form vorliegt.

10. Enantiomerstabiles R-Carprofen oder pharmazeutisch verträgliches Salz davon zur Verwendung für die Vorbeugung oder Verzögerung des Auftretens von Symptomen der Alzheimer Krankheit, wobei R-Carprofen oder dessen Salz als ein Arzneimittel, das im Wesentlichen kein S-Enantiomer von R-Carprofen enthält, bereitgestellt wird.

11. R-Carprofen nach Anspruch 10, wobei das Arzneimittel wie in einem der Ansprüche 2 bis 9 definiert ist.

## Revendications

1. Utilisation de R-carprofen énantiomériquement stable ou d'un sel pharmaceutiquement acceptable de celui-ci pour la fabrication d'un médicament pour la prévention ou le retardement de l'apparition des symptômes de la maladie d'Alzheimer, ledit médicament étant essentiellement exempt du S-énantiomère dudit R-carprofen.

2. Utilisation selon la revendication 1, le rapport dudit R-carprofen audit S-carprofen dans ledit médicament étant au moins de 90/10 en poids.

3. Utilisation selon la revendication 1 ou 2, dans laquelle le rapport dudit R-carprofen audit-S-carprofen est au moins de 99/1 en poids.

4. Utilisation selon une quelconque des revendications 1 à 3, dans laquelle ledit sel pharmaceutiquement acceptable dudit R-carprofen est un sel métallique ou un sel organique.

5. Utilisation selon la revendication 4, dans laquelle ledit sel métallique de R-carprofen est choisi parmi le groupe composé d'un sel de sodium, de potassium, de calcium, de magnésium, de lithium, d'aluminium et de zinc ou dans laquelle ledit sel organique de R-carprofen est choisi parmi le groupe composé d'un sel de lysine, de N,N'-bibenzyléthylènediamine, de chloroprocaïne, de choline, de diéthanolamine, d'éthylènediamine, de méglumine, de procaïne et de tris.

6. Utilisation selon une quelconque des revendications 1 à 5, dans laquelle une dose dudit médicament comprend environ 0,1 mg à 2000 mg dudit R-carprofen ou du sel de celui-ci.

7. Utilisation selon la revendication 4 ou 5, dans laquelle chaque dose dudit médicament comprend environ de 1 mg à 600 mg dudit R-carprofen ou du sel de celui-ci ;

8. Utilisation selon une quelconque des revendications 1 à 7, dans laquelle ledit médicament comprend en outre un support pharmaceutiquement acceptable.

9. Utilisation selon une quelconque des revendications 1 à 8, dans laquelle ledit médicament est présenté sous une forme adaptée à l'administration orale, transdermique, intraveineuse ou intrathécale.

10. R-carprofen énantiomériquement stable ou un sel pharmaceutiquement acceptable de celui-ci destiné à être utilisé pour la prévention ou le retardement de l'apparition des symptômes de la maladie d'Alzheimer, ledit R-carprofen ou sel de celui-ci étant mis à disposition comme médicament essentiellement exempt du S-énantiomère dudit R-carprofen.

11. R-carprofen selon la revendication 10, ledit médicament étant comme défini par l'une quelconque des revendications de 2 à 9.
